# EUROPEAN PATENT SPECIFICATION

(11) **EP 3 705 097 B1**
(45) Date of publication and mention of the grant of the patent: **29.06.2022**
(21) Application number: 18894517.4
(22) Date of filing: 11.12.2018
(51) Int. Cl.: A61F 13/49, A61F 13/511, A61F 13/513

(54) **ABSORBENT ARTICLE**
ABSORBIERENDER ARTIKEL
ARTICLE ABSORBANT

(30) Priority: 28.12.2017 JP 2017255004
(43) Date of publication of application: 09.09.2020
(73) Proprietor: Unicharm Corporation, Ehime 799-0111 (JP)
(72) Inventor: MAKI, Hideaki, Kanonji-shi, Kagawa 769-1602 (JP); INOUE, Takuya, Kanonji-shi, Kagawa 769-1602 (JP); OKUBO, Tetsuo, Kanonji-shi, Kagawa 769-1602 (JP); SHIMIZU, Noriko, Kanonji-shi, Kagawa 769-1602 (JP)
(74) Representative: Dolleymores
(86) International application number: PCT/JP2018/045481
(87) International publication number: WO 2019/131110

(56) References cited:
- EP-A1- 3 698 763
- WO-A1-2016/159095
- WO-A1-2016/159095
- WO-A1-2019/131111
- JP-A- 2014 100 157
- JP-U- H0 484 309
- US-A1- 2006 254 708

## Description

### [Technical Field]

The present invention relates to an absorbent article.

### [Background Art]

Pull-on disposable diapers are known as an example of an absorbent article. Patent Document 1 discloses a pull-on disposable diaper in which an absorbent main body is provided spanning between a front-panel exterior body and a back-panel exterior body, and the two widthwise side edges of the front-panel exterior body and the back-panel exterior body are joined to each other so as to form a waist opening. In the front-panel exterior body and the back-panel exterior body, waist elastic members are fixed stretching between an inner sheet and an outer sheet (non-stretchy sheets).

### [Citation List]

### [Patent Literature]

[Patent Document 1] Japanese Patent Application Publication No. 2013-138795

WO 2016/159095 A1 relates to a pants-type disposable diaper capable of strongly tightening the edge portion of an opening. To achieve this, a laminated portion is folded over in the waist opening and/or in the leg opening region. This laminated portion consists of an elastic film sandwiched between two sheet layers.

### [Summary of Invention]

### [Technical Problem]

In Patent Document 1, string-like elastic members are given as an example of the waist elastic members, but using stretchy sheets instead makes it possible for the front-panel exterior body and the back-panel exterior body (waist portions) to come into close planar contact with the wearer. This therefore makes it possible to suppress the case where elastic members leave marks on the wearer's skin.

Also, appropriately lowering the stretching force in the waist-opening-side end portions of the stretchy sheet allows the waist opening to be easily spread open when putting the diaper on, and allows the diaper to be put on easily. However, if the stretching force in the crotch-side end portions of the stretchy sheet is also lowered to match the waist opening side, there is a risk of positional displacement of the absorbent main body.

The present invention was achieved in light of conventional problems such as that described above, and an aspect of the present invention is to provide an absorbent article that can suppress positional displacement of the absorbent main body while also maintaining the ability of the waist opening to be spread open easily.

### [Solution to Problem]

The present invention provides the absorbent article of independent claim 1. The dependent claims specify preferred but optional features.

A main aspect of the present invention for achieving the above-described aspect is an absorbent article having a vertical direction and a lateral direction,
the absorbent article including:
an absorbent main body;
a first waist portion; and
a second waist portion,
   the first waist portion including a stretchy sheet and a non-stretchy sheet,
   the stretchy sheet being capable of stretching and contracting in the lateral direction,
   the non-stretchy sheet being overlaid on the stretchy sheet,
   a lower end portion of the stretchy sheet being folded upward,
   a number of layers of the stretchy sheet being greater in the lower end portion of the stretchy sheet than in an upper end portion of the stretchy sheet, wherein
   the stretchy sheet is located on a skin side in a thickness direction of the first waist portion with respect to the absorbent main body, and
in the lower end portion of the stretchy sheet,
   a folded portion of the stretchy sheet is overlapped with the absorbent main body in the vertical direction.

Features of the present invention other than the above will become clear by reading the description of the present specification with reference to the accompanying drawings.

### [Advantageous Effects of Invention]

According to the present invention, it is possible to provide an absorbent article that can suppress positional displacement of the absorbent main body while also maintaining the ability of the waist opening to be spread open easily.

### [Brief Description of the Drawings]

FIG. 1 is a perspective view of a pull-on disposable diaper 1 (hereinafter, "diaper").
FIG. 2 is a plan view of the diaper 1 in an unfolded and stretched state.
FIG. 3 is a cross-sectional view taken along a line I-I in FIG. 2.
FIG. 4 is a cross-sectional view of a front waist portion 20 in an in-production state in a reference example which does not form part of the present invention.
FIG. 5 is a cross-sectional view of the front waist portion 20 of the reference example in a product state.
FIG. 6 is a plan view of the front waist portion 20 shown in FIG. 4.
FIGS. 7A and 7B are cross-sectional views of a variation of the front waist portion 20 in a reference example which does not form part of the present invention.
FIG. 8A is a cross-sectional view of a variation of the front waist portion 20 in a reference example which does not form part of the present invention.
FIG. 8B is a cross-sectional view of a variation of the front waist portion 20 according to the present invention.
FIG. 9A is a cross-sectional view of a variation of the front waist portion 20 in a reference example which does not form part of the present invention.
FIG. 9B is a cross-sectional view of a variation of the front waist portion 20 according to the present invention.
FIG. 10A is a cross-sectional view of a variation of the front waist portion 20 in a reference example which does not form part of the present invention.
FIG. 10B is a cross-sectional view of a variation of the front waist portion 20 according to the present invention.
FIG. 11A is a cross-sectional view of a variation of the front waist portion 20 in a reference example which does not form part of the present invention.
FIG. 11B is a cross-sectional view of a variation of the front waist portion 20 according to the present invention.

### [Description of Embodiments]

At least the following matters will become clear with the description of this specification and the attached drawings. An absorbent article having a vertical direction and a lateral direction,
the absorbent article including:
an absorbent main body;
a first waist portion; and
a second waist portion,
   the first waist portion including a stretchy sheet and a non-stretchy sheet,
   the stretchy sheet being capable of stretching and contracting in the lateral direction,
   the non-stretchy sheet being overlaid on the stretchy sheet,
   a lower end portion of the stretchy sheet being folded upward,
   a number of layers of the stretchy sheet being greater in the lower end portion of the stretchy sheet than in an upper end portion of the stretchy sheet.

According to this absorbent article, there are fewer layers of the stretchy sheet in the upper end portion thereof, thus making it possible to maintain the ease of spreading the waist opening. Also, there are more layers of the stretchy sheet in the lower end portion thereof, thus making it possible to suppress positional displacement of the absorbent main body.

In the absorbent article of the present invention,
the stretchy sheet is located on a skin side in a thickness direction of the first waist portion with respect to the absorbent main body, and
in the lower end portion of the stretchy sheet,
   a folded portion of the stretchy sheet is overlapped with the absorbent main body in the vertical direction.

According to this absorbent article, the upper end (edge) of the absorbent main body can be covered by the stretchy sheet on the skin side, and it is possible to suppress the case where the wearer feels an unpleasant portion.

In such an absorbent article,
the absorbent main body includes an absorbent core configured to absorb an excreted fluid, and
the folded portion of the stretchy sheet is not overlapped with the absorbent core in the vertical direction.

According to this absorbent article, the stretching force in the folded portion of the stretchy sheet is less likely to be inhibited by the stiffer absorbent core. For this reason, positional displacement of the absorbent main body can be suppressed.

In such an absorbent article,
the folded portion of the stretchy sheet is provided extending across an upper end of the absorbent main body in the vertical direction.

According to this absorbent article, it is possible to gradually vary the number of material layers in the vicinity of the upper end of the absorbent main body, and it is possible to suppress the case where the wearer feels an unpleasant portion.

In such an absorbent article,
the absorbent main body includes an absorbent core configured to absorb an excreted fluid, and
the folded portion of the stretchy sheet is overlapped with the absorbent core in the vertical direction.

According to this absorbent article, in the case where the stretchy sheet is located on the non-skin side of the absorbent main body, the folded portion of the stretchy sheet can bring the absorbent core into close contact with the wearer from the non-skin side, and it is possible to suppress positional displacement of the absorbent main body. Also, in the case where the stretchy sheet is located on the skin side of the absorbent main body, the upper end (edge) of the absorbent core can be covered by the stretchy sheet on the skin side, and it is possible to suppress the case where the wearer feels an unpleasant portion.

In such an absorbent article,
the folded portion of the stretchy sheet is provided extending across an upper end of the absorbent core in the vertical direction.

According to this absorbent article, it is possible to gradually vary the number of material layers in the vicinity of the upper end of the absorbent core, and it is possible to suppress the case where the wearer feels an unpleasant portion.

In such an absorbent article,
in a state where the lower end portion of the stretchy sheet is folded back,
   two lateral side end portions of the first waist portion are welded to two lateral side end portions of the second waist portion.

According to this absorbent article, it is possible to maintain the folded state of the lower end portion of the stretchy sheet.

### Embodiment

The following describes an embodiment of an absorbent article according to the present invention by way of example of a pull-on disposable diaper for infants. Note that the absorbent article according to the present invention is not limited to being a disposable diaper for infants, and is also applicable to a disposable diaper for adults and sanitary shorts, for example. Further, it is also applicable not only to a pull-on disposable diaper, but also to a tape-type disposable diaper, for example.

### Configuration of pull-on disposable diaper 1

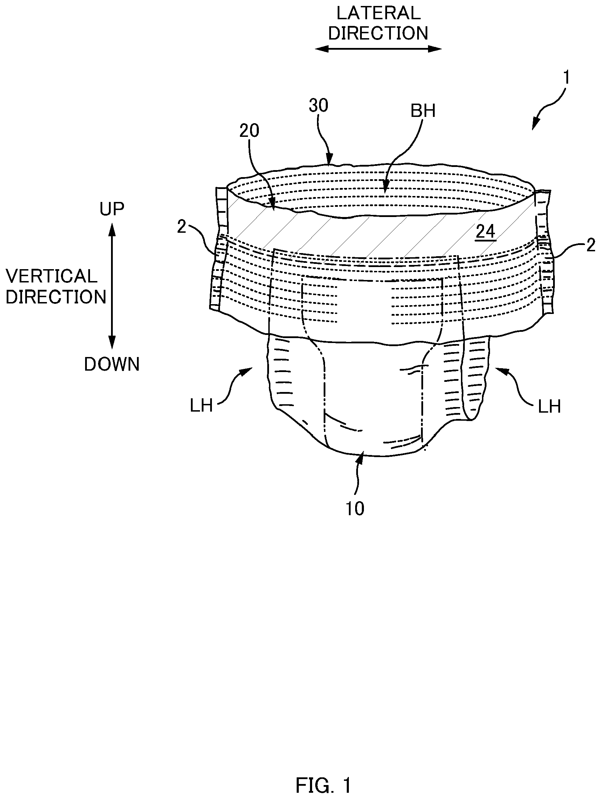

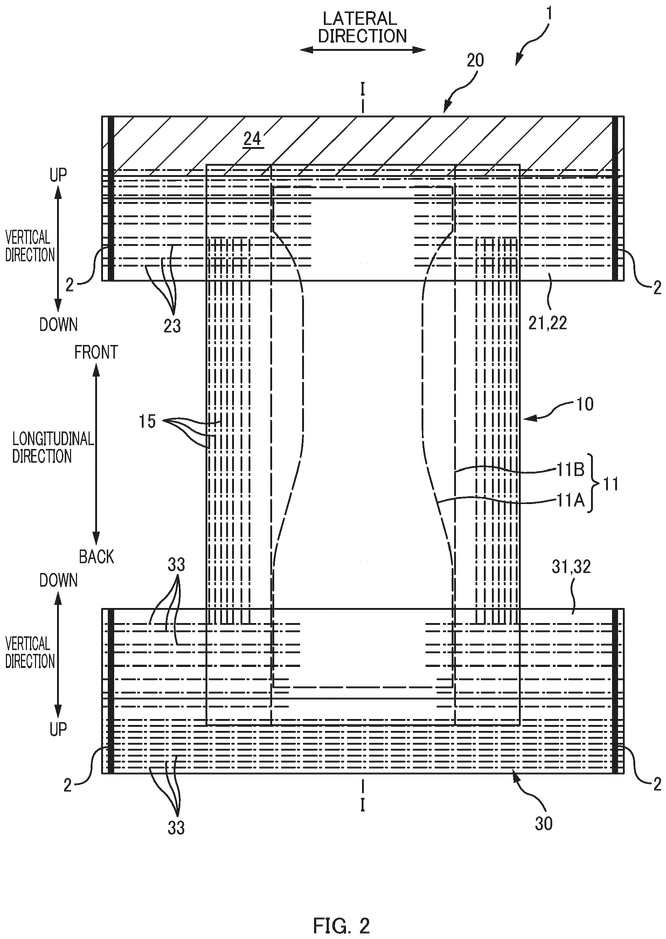

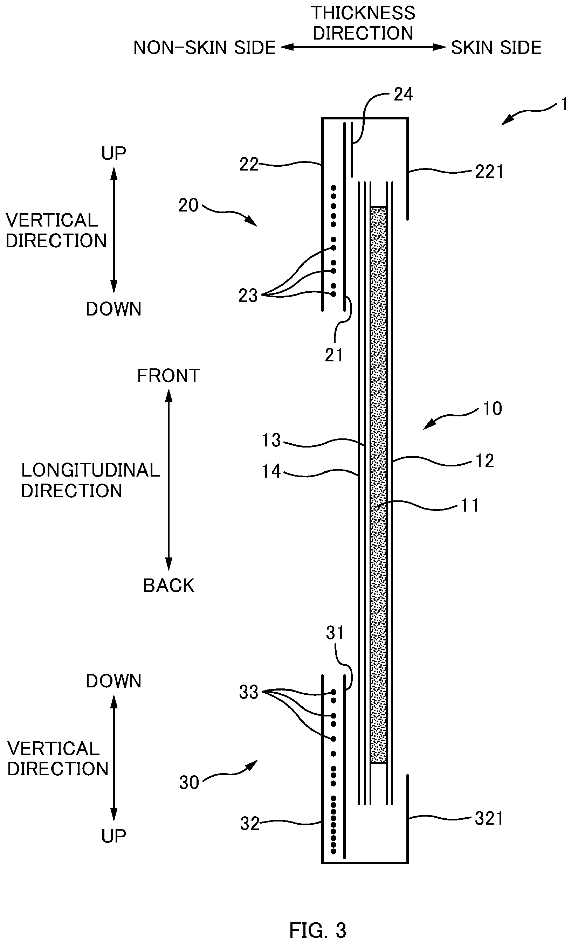

FIG. 1 is a perspective view of a pull-on disposable diaper 1 (hereinafter, "diaper"). FIG. 2 is a plan view of the diaper 1 in an unfolded and stretched state. FIG. 3 is a cross-sectional view taken along a line I-I in FIG. 2.

In the underpants-shaped state shown in FIG. 1, the diaper 1 has a vertical direction and a lateral direction, and includes a waist opening BH and a pair of leg openings LH. With respect to the vertical direction, the waist opening BH side is the upper side, and the crotch side is the lower side. Also, as shown in FIG. 3, the thickness direction refers to the direction in which constituent members of the diaper 1 are overlaid on each other, the skin side refers to the side that comes into contact with the wearer in the thickness direction, and the non-skin side refers to the side that does not come into contact with the wearer in the thickness direction.

The diaper 1 also includes an absorbent main body 10, a front waist portion 20, and a back waist portion 30, which are three members that are rectangular in a plan view. The front waist portion 20 (first waist portion) comes into contact with the front region of the wearer, and the back waist portion 30 (second waist portion) comes into contact with the back region of the wearer.

When the diaper 1 is in the unfolded state shown in FIG. 2, the front waist portion 20 and the back waist portion 30 are arranged such that the longitudinal directions thereof conform to the lateral direction of the diaper 1. The end portion of the absorbent main body 10 on the longitudinal one side thereof is arranged in the central portion of the front waist portion 20 with respect to the lateral direction, and the end portion of the absorbent main body 10 on the longitudinal other side thereof is arranged in the central portion of the back waist portion 30 with respect to the lateral direction.

When the diaper 1 is in the unfolded state shown in FIG. 2, the absorbent main body 10 is folded one time at approximately the longitudinal center such that the longitudinal direction of the absorbent main body 10 conforms to the vertical direction of the diaper 1. The two side end portions of the front waist portion 20 in the lateral direction of the diaper 1 are joined to the two side end portions of the back waist portion 30 with use of heat welding, ultrasonic welding, or the like to form a pair of joining regions 2, thus obtaining the diaper 1 in the underpants-shaped state shown in FIG. 1.

As shown in FIG. 3, the absorbent main body 10 includes an absorbent body 11, a liquid-permeable top sheet 12 arranged on the skin side of the absorbent body 11, a liquid-impermeable back sheet 13 arranged on the non-skin side of the absorbent body 11, and an exterior sheet 14 arranged on the non-skin side of the back sheet 13.

The absorbent body 11 includes an absorbent core 11A that absorbs and holds an excreted fluid such as urine, and a liquid-permeable core-wrapping sheet 11B that covers the absorbent core 11A. For example, the absorbent core 11A is constituted by liquid-absorbent fibers such as pulp containing a superabsorbent polymer (SAP), which are molded into a predetermined shape.

Also, as shown in FIG. 2, leg elastic members 15 are provided in two side portions of the absorbent main body 10 in the lateral direction, thus allowing the diaper 1 to fit to the wearer's legs. For example, the leg elastic members 15 are fixed in a state of being stretched in the longitudinal direction of the absorbent main body 10 between two layers of the exterior sheet 14 that has been folded inward in the lateral direction. The leg elastic members 15 are string-like elastic members, stretchy sheets, or the like.

As shown in FIG. 3, the front waist portion 20 and the back waist portion 30 respectively include inner layer sheets 21 and 31, outer layer sheets 22 and 32 overlaid on the inner layer sheets 21 and 31, and multiple string-like elastic members 23 and 33.

The outer layer sheets 22 and 32 are arranged on the non-skin side of the inner layer sheets 21 and 31. The inner layer sheets 21 and 31 and the outer layer sheets 22 and 32 are arranged on the non-skin side of the absorbent main body 10. It should be noted that upper end portions 221 and 321 of the outer layer sheets 22 and 32 are folded downward so as to cover the longitudinal end portions of the absorbent main body 10 from the skin side.

The inner layer sheets 21 and 31 and the outer layer sheets 22 and 32 are each a non-stretchy sheet that has substantially no stretchability in the lateral direction of the diaper 1. The inner layer sheets 21 and 31 and the outer layer sheets 22 and 32 can be constituted by an SMS nonwoven fabric sheet (spunbond/meltblown/spunbond nonwoven fabric sheet), a spunbond nonwoven fabric sheet, an air-through nonwoven fabric sheet, a plastic sheet, a porous plastic sheet, or a laminate sheet including layers of any of the same.

The string-like elastic members 23 and 33 are arranged side-by-side in the vertical direction between the inner layer sheets 21 and 31 and the outer layer sheets 22 and 32, and are fixed in a state of being stretched in the lateral direction. The front waist portion 20 and the back waist portion 30 can thus stretch and contract in the lateral direction so as to fit to the wearer's waist. The string-like elastic members 23 and 33 can be constituted by a string-like elastic member made of rubber, spandex, or the like.

In the back waist portion 30, the string-like elastic members 33 are arranged within a range from the upper end portion to the lower end portion. However, the upper end portion of the front waist portion 20 is not provided with the string-like elastic members 23, and instead is provided with a stretchy sheet 24 that has stretchability in the lateral direction. Note that although the lower end portion of the stretchy sheet 24 is overlapped with a string-like elastic member 23 in the vertical direction in the present embodiment, this overlapping is not required.

According to this configuration in which the upper end portion of the front waist portion 20 is not provided with the string-like elastic members 23 but is provided with the stretchy sheet 24, the upper end portion of the front waist portion 20 comes into close surface contact with the wearer. This therefore suppresses localized constriction, and can suppress the formation of marks by stretching/contracting members. In particular, an infant has a bulging stomach region, and therefore it is preferable to suppress constriction in the front waist portion 20.

Also, stretching force in the upper end portions of the waist portions 20 and 30 largely influences the ability to easily spread open the waist opening BH when the diaper 1 is to be put on. The stretchy sheet 24 has a characteristic of easily stretching from the natural state, but not easily contracting from the stretched state. For this reason, by providing the stretchy sheet 24 in the upper end portion of the front waist portion 20, it is possible to make it easier to spread open the waist opening BH when putting on the diaper 1, and also prevent constriction of the front region after the waist portions 20 and 30 have been fitted to the wearer.

The stretchy sheet 24 can be constituted by a stretchy-fiber nonwoven-fabric sheet that is manufactured by any of various known manufacturing methods, such as a spunbond nonwoven fabric sheet, an air-through nonwoven fabric sheet, a needle punch nonwoven fabric sheet, and the like, which are constituted by elastic fibers having a mass of 10 to 40 g/m², or more preferably 15 to 30 g/m². The raw material constituting the elastic fibers is a thermoplastic elastomer, rubber, or the like. Particularly in the case where the raw material is a thermoplastic elastomer, it is possible to perform melt spinning with use of an extruder similarly to normal thermoplastic resin. The thus-obtained fibers can be easily heat welded, which is favorable for a stretchy-fiber nonwoven-fabric sheet. Examples of thermoplastic elastomers include styrene elastomer, olefin elastomer, polyester elastomer, and polyurethane elastomer. It is possible to use any of the above on their own, or a combination of two or more. The stretchy sheet need only exhibit elasticity in at least the lateral direction, and may exhibit elasticity in two or more directions. The stretch factor of the stretchy sheet 24 in the lateral direction is preferably a factor of approximately 1.2 to 3.0.

### Number of layers of stretchy sheet 24

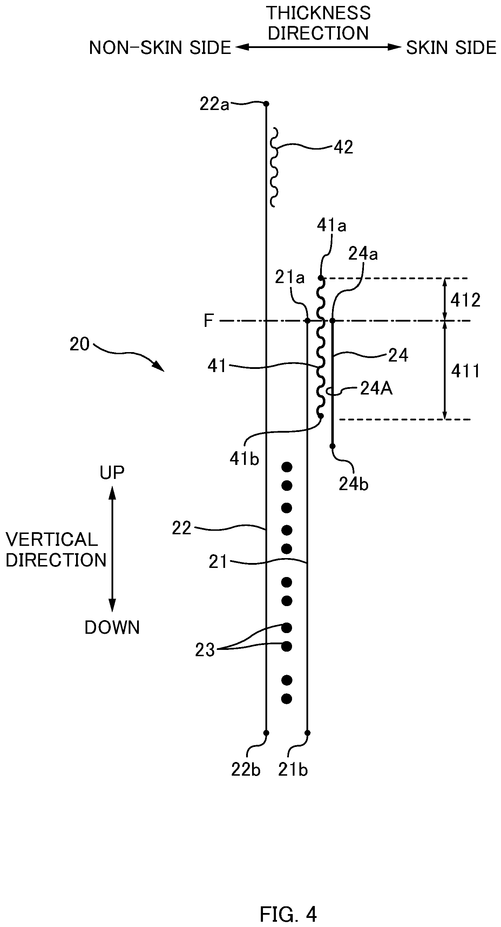

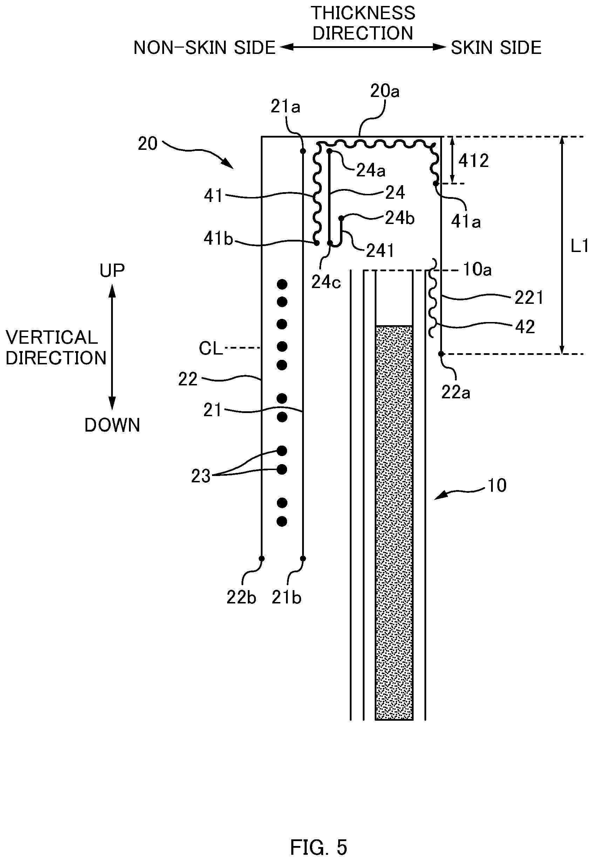

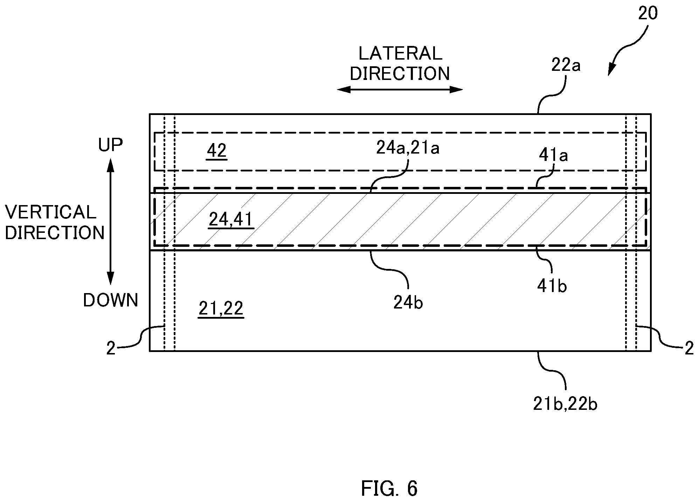

FIG. 4 is a cross-sectional view of the front waist portion 20 in an in-production state in a reference example. FIG. 5 is a cross-sectional view of the front waist portion 20 in a product state in the reference example. FIG. 6 is a plan view of the front waist portion 20 shown in FIG. 4. Note that the configuration of the front waist portion 20 is shown schematically in FIGS. 4 and 5, and the front waist portion 20 has been drawn thicker than in actuality. This similarly applies to later-described cross-sectional views as well. Also, the string-like elastic members 23 are not shown in FIG. 6.

As previously described, the front waist portion 20 includes the inner layer sheet 21, the outer layer sheet 22, and the stretchy sheet 24. In the in-production state (FIG. 4), the outer layer sheet 22, the inner layer sheet 21, and the stretchy sheet 24 are overlaid on each other in this order starting from the non-skin side in the thickness direction.

As shown in FIG. 6, the inner layer sheet 21, the outer layer sheet 22, and the stretchy sheet 24 respectively have first ends 21a, 22a, 24a and second ends 21b, 22b, and 24b that extend in the lateral direction. In the product state (FIG. 5), the first ends 21a, 22a, and 24a of the sheets are at positions higher than the second ends 21b, 22b, and 24b.

The inner layer sheet 21 and the outer layer sheet 22 are joined together using an adhesive (not shown) applied therebetween. The inner layer sheet 21 and the outer layer sheet 22 correspond to non-stretchy sheets of the present invention. Hereinafter, the inner layer sheet 21 and the outer layer sheet 22 are together also called non-stretchy sheets.

In the in-production state (FIG. 4), the position of the first end 21a of the inner layer sheet 21 and the position of the first end 24a of the stretchy sheet 24 are aligned with each other in the vertical direction. The outer layer sheet 22 extends to a position higher than the inner layer sheet 21 and the stretchy sheet 24.

In the product state (FIG. 5), the outer layer sheet 22 has been folded downward at a folding position F that corresponds to the position of the first ends 21a and 24a of the inner layer sheet 21 and the stretchy sheet 24. A folded portion 221 of the outer layer sheet 22 covers a longitudinal end portion of the absorbent main body 10 on the skin side. Also, the folded portion 221 of the outer layer sheet 22 is fixed to the absorbent main body 10 using an adhesive 42.

Also, an adhesive 41 that is a hot-melt adhesive or the like has been applied between the non-stretchy sheets (inner layer sheet 21 and outer layer sheet 22) and the stretchy sheet 24. The stretchy sheet 24 is fixed to the non-stretchy sheets 21 and 22 by the adhesive 41. As shown in FIG. 6, the application region where the adhesive 41 is applied is a rectangular region in a plan view. Hereinafter, the application region where the adhesive 41 is applied is also called a fixing portion 41.

Note that the adhesive 41 may be applied in only portions of the application region where the adhesive 41 is applied, or the adhesive 41 may be applied to the entirety of the application region. The application pattern of the adhesive 41 can be a known pattern such as a stripe pattern, a spiral pattern, or an Ω pattern. Also, the planar shape of the application region where the adhesive 41 is applied is not limited to being a rectangular shape. Moreover, the fixing portion may be a welding region in which the stretchy sheet 24 is fixed to the non-stretchy sheets 21 and 22 through heat welding, ultrasonic welding, or the like.

As shown in FIG. 6, the fixing portion 41 has a first end 41a and a second end 41b that extend in the lateral direction. In the product state (FIG. 5), the first end 41a of the fixing portion 41 is at a higher position than the second end 41b is.

Also, the fixing portion 41 extends across the first end 24a of the stretchy sheet 24 in the vertical direction. Specifically, in the in-production state (FIG. 4), the fixing portion 41 is located between the stretchy sheet 24 and the inner layer sheet 21, and extends beyond the first end 24a of the stretchy sheet 24 to a position on the skin-side surface of the outer layer sheet 22. For this reason, in the product state (FIG. 5), as the outer layer sheet 22 is folded, a portion of the fixing portion 41 is also folded downward, and the fixing portion 41 covers the first end 24a of the stretchy sheet 24 from above. Accordingly, in the product state (FIG. 5), the fixing portion 41 extends across the first end 24a of the stretchy sheet 24 in the vertical direction.

Due to the fixing portion 41 being provided in this manner, a portion of the stretchy sheet 24 that is on the first end 24a side and extends up to the first end 24a is fixed to the non-stretchy sheets 21 and 22. For this reason, it is possible to suppress the case where, during production, the first-end-24aside portion of the stretchy sheet 24 curls and is folded downward due to the contractive force of the stretchy sheet 24.

Note that the position of the first end 41a of the fixing portion 41 and the position of the first end 24a of the stretchy sheet 24 may be aligned with each other in the vertical direction. However, providing the fixing portion 41 so as to extend across the first end 24a of the stretchy sheet 24 makes it possible to more reliably suppress curling of the first-end-24a-side portion of the stretchy sheet 24.

Also, the method for manufacturing the diaper 1 does not have a step of folding the first-end-24a-side portion of the stretchy sheet 24. Accordingly, in the upper end portion of the stretchy sheet 24, the stretchy sheet 24 is not folded downward, and there is one layer of the stretchy sheet 24.

Also, the fixing portion 41 does not extend across the second end 24b of the stretchy sheet 24 in the vertical direction. Specifically, when the stretchy sheet 24 is fixed to the non-stretchy sheets 21 and 22 (FIG. 4), the second end 41b of the fixing portion 41 is located at a position higher than the second end 24b of the stretchy sheet 24. In other words, the fixing portion 41 is not provided at a portion of the stretchy sheet 24 on the second end 24b side. For this reason, during production, a portion of the stretchy sheet 24 that is on the second end 24b side curls due to the contractive force of the stretchy sheet 24.

It is thought that this curling occurs due to localized contraction of the stretchy sheet 24 caused by slight differences in how tension acts on the stretchy sheet 24 during transporting after the stretchy sheet 24 has been fixed to the non-stretchy sheets 21 and 22 in the processing of manufacturing the diaper 1. Curling is also thought to occur in the case where the stretchy sheet 24 is temporarily stretched in the width direction, which intersects a direction in which the stretchy sheet 24 extends.

Also, the second-end-24b-side portion of the stretchy sheet 24 is overlapped with the absorbent main body 10. For this reason, in the product state, the second-end-24b-side portion of the stretchy sheet 24 not only curls and rises in the thickness direction, but is also folded upward. Specifically, the second-end-24b-side portion of the stretchy sheet 24 is folded upward at the lower end 41b of the fixing portion 41.

Note that the stretchy sheet 24 may be folded such that a fold line that extends in the lateral direction is formed, or may be folded such that the lower end of the stretchy sheet 24 curves (i.e., curls) as shown in FIG. 5.

Also, the method for manufacturing the diaper 1 does not have a step of folding the second-end-24b-side portion of the stretchy sheet 24. Accordingly, in the lower end portion of the stretchy sheet 24, there are two layers of the stretchy sheet 24.

In other words, the number of layers of the stretchy sheet 24 is greater in the lower end portion of the stretchy sheet 24 than in the upper end portion thereof. As the number of layers of the stretchy sheet 24 increases due to being folded, the stretching force in the lateral direction increases by an amount commensurate to the folded portion of the stretchy sheet 24.

For this reason, the stretching force in the lateral direction is smaller in the upper end portion of the stretchy sheet 24, that is to say in the end portion on the waist opening BH side, than in the lower end portion. In other words, in the upper end portion of the stretchy sheet 24, curling of the stretchy sheet 24 is suppressed, and the stretching force is appropriately suppressed as designed. This therefore makes it possible to maintain the ability to easily spread open the waist opening BH when putting on the diaper 1, and to prevent constriction of the wearer's stomach region.

In particular, the ease of spreading of the waist opening BH is greatly influenced by the stretching force of the upper end portions of the waist portions 20 and 30. For this reason, in the case where the stretchy sheet 24 is provided in the upper end portion of the front waist portion 20 as in the diaper 1 of the present embodiment, it is preferable to reduce the number of layers in the upper end portion of the stretchy sheet 24 in particular. This therefore makes it even easier to spread open the waist opening BH.

Note that the stretchy sheet 24 being provided in the upper end portion of the front waist portion 20 refers to the case where at least a portion of the stretchy sheet 24 is located higher than the vertical center CL of the front waist portion 20 (see FIG. 5). More preferably, the entirety of the stretchy sheet 24 is located higher than the vertical center CL of the front waist portion 20.

On the other hand, the lower end portion of the stretchy sheet 24, that is to say the end portion on the crotch side, has a greater stretching force in the lateral direction than the upper end portion has. Specifically, the stretching force has been set greater in the portion of the front waist portion 20 that is overlapped with the absorbent main body 10. This therefore makes it possible to suppress positional displacement of the absorbent main body 10. Specifically, it is possible to suppress positional displacement of the absorbent main body 10 caused by movement of the wearer, and to suppress drooping of the absorbent main body 10 caused by the absorption of excreted fluid. This therefore makes it possible to maintain the fit of the absorbent main body to the wearer and to reduce the leakage of excrement. Also, the wearer does not feel discomfort and can walk more easily, for example.

Moreover, the stretching force in the lower end portions of the waist portions 20 and 30 has a smaller influence on the ease of spreading the waist opening BH than the stretching force in the upper end portions does. Accordingly, the ease of spreading of the waist opening BH can be maintained even if there are two layers of the stretchy sheet 24 in the lower end portion.

Also, a configuration is possible in which the fixing portion 41 is provided extending across the second end 24b of the stretchy sheet 24 in the vertical direction, and the method for manufacturing the diaper 1 may include a folding step of folding the second-end-24b-side portion of the stretchy sheet 24. Even in this case, the number of layers of the stretchy sheet 24 in the lower end portion can be set greater than in the upper end portion. It should be noted that by utilizing the contractive force of the stretchy sheet 24 and folding the second-end-24b-side portion of the stretchy sheet 24 as described above, it is possible to increase the stretching force in the lower end portion of the stretchy sheet 24 without an increase in the complexity of the method for manufacturing the diaper 1.

Also, in the front waist portion 20 in the reference example of FIG. 5, in the lower end portion of the stretchy sheet 24, the portion of the stretchy sheet 24 that has been folded back (hereinafter, also called a folded portion 241) is located higher than an upper end 10a of the absorbent main body 10. In other words, the folded portion 241 is not overlapped with the absorbent main body 10 in the vertical direction. Note that the folded portion 241 is specifically the portion that extends from a portion 24c of the stretchy sheet 24 that is contact with the second end 41b of the fixing portion 41 to the second end 24b of the stretchy sheet 24.

In the case of this reference example, the folded portion 241 can bring the portion of the front waist portion 20 in the vicinity of the absorbent main body 10 into close contact with the wearer's body, thus suppressing positional displacement of the absorbent main body 10 caused by positional displacement of the front waist portion 20. This therefore makes it possible to maintain the fit of the absorbent main body 10 to the wearer and reduce the leakage of excrement. Also, the stretching force of the folded portion 241 is not likely to be inhibited by the absorbent main body 10 that has a higher stiffness. This therefore makes it possible to suppress positional displacement of the absorbent main body 10.

Also, the pull-on diaper 1 has the pair of joining regions 2 in which the two lateral side end portions of the front waist portion 20 are joined to the two lateral side end portions of the back waist portion 30 by welding. As shown in the reference example of FIG. 6, it is preferable that the stretchy sheet 24 is provided extending outward in the lateral direction with respect to the positions where the joining regions 2 are formed. Also, it is preferable that the joining regions 2 are formed in a state where the second-end-24b-side portion of the stretchy sheet 24 is folded upward in the lower end portion of the stretchy sheet 24. According to this configuration, the folded state of the second-end-24b-side portion of the stretchy sheet 2 is maintained, and it is possible to suppress positional displacement of the absorbent main body 10 while the diaper 1 is put on. This therefore makes it possible to maintain the fit of the absorbent main body 10 to the wearer and reduce the leakage of excrement.

### Variations

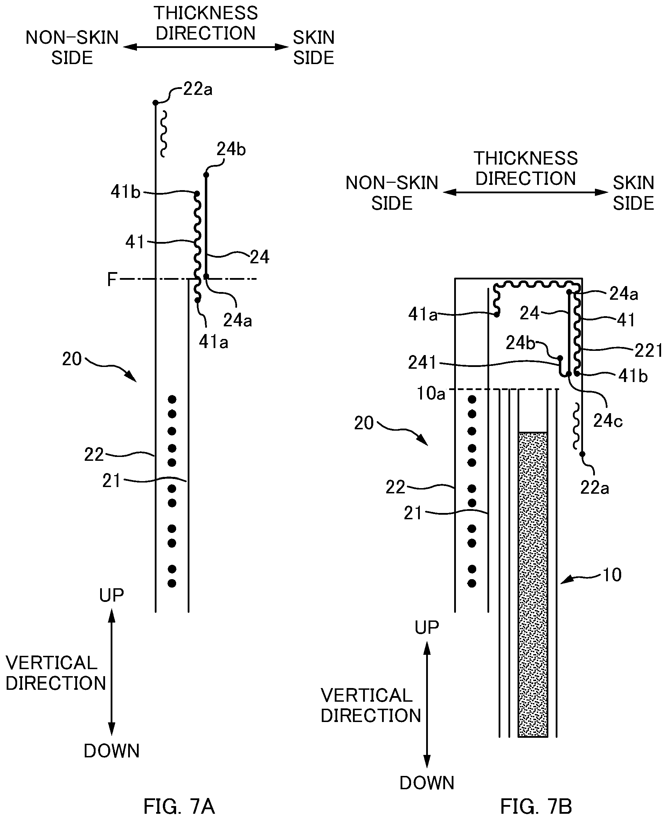

FIGS. 7A and 7B are cross-sectional views of a variation of the front waist portion 20 in a reference example. FIG. 7A is a diagram showing the front waist portion 20 in an in-production state, and FIG. 7B is a diagram showing the front waist portion 20 in the product state. As shown in FIG. 7B, in the front waist portion 20, the stretchy sheet 24 may be fixed by a fixing portion 41 to a portion 221 of the outer layer sheet 22 that has been folded downward.

In the case of this reference example, in the in-production state (FIG. 7A), the lower end 24a of the stretchy sheet 24 is the first end 24a of the stretchy sheet 24, and the fixing portion 41 extends downward beyond the lower end 24a of the stretchy sheet 24. According to this configuration, in the product state (FIG. 7B), the stretchy sheet 24 is not likely to curl in the upper end portion thereof, and there is one layer of the stretchy sheet 24 in the upper end portion thereof.

Also, in the in-production state of this reference example (FIG. 7A), the upper end 24b of the stretchy sheet 24 is the second end 24b of the stretchy sheet 24, and the fixing portion 41 is not provided at the second-end-24b-side portion of the stretchy sheet 24. According to this configuration, in the product state (FIG. 7B), the stretchy sheet 24 is folded upward in the lower end portion thereof, and there are two layers of the stretchy sheet 24 in the lower end portion thereof.

In the case of the reference example of FIG. 7B as well, there are more layers of the stretchy sheet 24 in the lower end portion of the stretchy sheet 24 than in the upper end portion. This therefore makes it possible to suppress positional displacement of the absorbent main body 10 while also maintaining the ease of spreading the waist opening BH. This therefore makes it possible to maintain the fit of the absorbent main body 10 to the wearer and reduce the leakage of excrement.

Also, although there is one layer of the stretchy sheet 24 in the upper end portion thereof and two layers in the lower end portion thereof in the reference examples of FIGS. 5 and 7B described above, there are no particular limitations on the number of layers. For example, a configuration is possible in which there is one layer of the stretchy sheet 24 in the upper end portion thereof and three layers in the lower end portion thereof, and a configuration is possible in which there are two layers of the stretchy sheet 24 in the upper end portion thereof and three layers in the lower end portion thereof.

Also, although not shown, a configuration is possible in which the fixing portion 41 is not provided at the first-end-24a-side portion of the stretchy sheet 24, and the first-end-24a-side portion of the stretchy sheet 24 curls and is folded in the vertical direction. Also, the method for manufacturing the diaper 1 may include a step of folding the first-end-24a-side portion of the stretchy sheet 24, or a step of folding the second-end-24b-side portion of the stretchy sheet 24. In these cases as well, it is sufficient that the number of layers of the stretchy sheet 24 is greater in the lower end portion of the stretchy sheet 24 than in the upper end portion thereof.

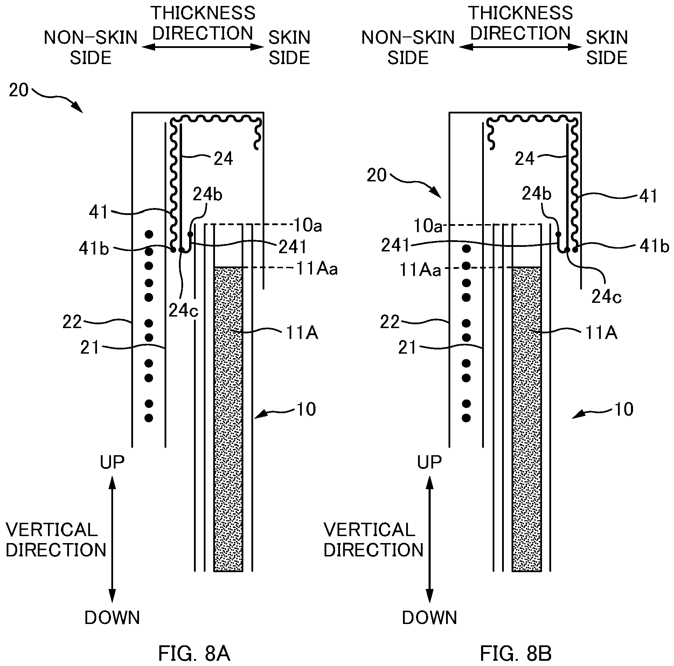

FIGS. 8 to 11 are cross-sectional views of variations of the front waist portion 20, and illustrate the position of the folded portion 241 of the stretchy sheet 24 in the vertical direction. Note that in FIGS. 8A, 9A, 10A, and 11A, the folded portion 241 is located on the non-skin side of the absorbent main body 10. These are therefore reference examples which do not form part of the present invention. On the other hand, in FIGS. 8B, 9B, 10B, and 11B, the folded portion 241 is located on the skin side of the absorbent main body 10, in accordance with the present invention.

In the front waist portion 20 in FIGS. 8A and 8B, the folded portion 241 of the stretchy sheet 24 is located lower than the upper end 10a of the absorbent main body 10, and furthermore is located higher than an upper end 11Aa of the absorbent core 11A. In other words, the folded portion 241 is overlapped with the upper end portion of the absorbent main body 10 (the top sheet 12, the back sheet 13, the exterior sheet 14, and the core-wrapping sheet 11B) in the vertical direction, but is not overlapped with the absorbent core 11A.

Also, the folded portion 241 in the reference example of FIG. 8A is located on the non-skin side of the absorbent main body 10. Accordingly, the folded portion 241 can bring the upper end portion of the absorbent main body 10 into close contact with the wearer from the non-skin side, and positional displacement of the absorbent main body 10 can be suppressed. This therefore makes it possible to maintain the fit of the absorbent main body 10 to the wearer and reduce the leakage of excrement.

On the other hand, the folded portion 241 in FIG. 8B is located on the skin side of the absorbent main body 10. In this case as well, the portion of the front waist portion 20 that is overlapped with the upper end portion of the absorbent main body 10 can be brought into close contact with the wearer by the folded portion 241, and positional displacement of the absorbent main body 10 can be suppressed. Also, in FIG. 8B, the stretchy sheet 24 covers the skin side of the upper end 10a of the absorbent main body 10 along with the outer layer sheet 22. Accordingly, the wearer is less likely to feel the upper end 10a (edge) of the absorbent main body 10, and it is possible to suppress the case where the wearer feels an unpleasant portion.

Also, the region of the absorbent main body 10 where the absorbent core 11A is provided has a higher stiffness than the region where the absorbent core 11A is not provided. Accordingly, in the case shown in FIGS. 8A and 8B, the stretching force of the folded portion 241 is less likely to be inhibited by the absorbent core 11A that has a higher stiffness.

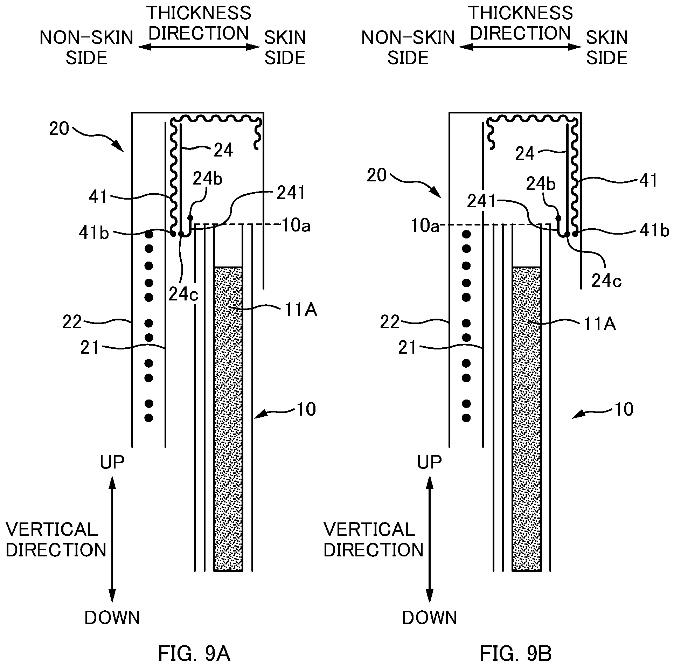

In the front waist portion 20 in FIGS. 9A and 9B, the folded portion 241 of the stretchy sheet 24 extends across the upper end 10a of the absorbent main body 10 in the vertical direction. In this case, it is possible to gradually vary the number of material layers in the vicinity of the upper end 10a of the absorbent main body 10.

Specifically, the number of material layers can increase from the upper side to the lower side, that is to say, there is a region having one layer of the stretchy sheet 24, a region having two layers of the stretchy sheet 24 (the folded portion 241), and a region in which the absorbent main body 10 is overlaid on the portion having two layers of the stretchy sheet 24 (the folded portion 241). Accordingly, it is possible to reduce a height difference and a stiffness difference between the region having the absorbent main body 10 and the region not having it, and it is possible to suppress the case where the wearer feels an unpleasant portion.

In particular, in FIG. 9B, the folded portion 241 of the stretchy sheet 24 covers the skin side of the upper end 10a of the absorbent main body 10. Accordingly, even more so than in the case shown in FIG. 8B, the wearer is less likely to feel the upper end 10a (edge) of the absorbent main body 10, and it is possible to suppress the case where the wearer feels an unpleasant portion.

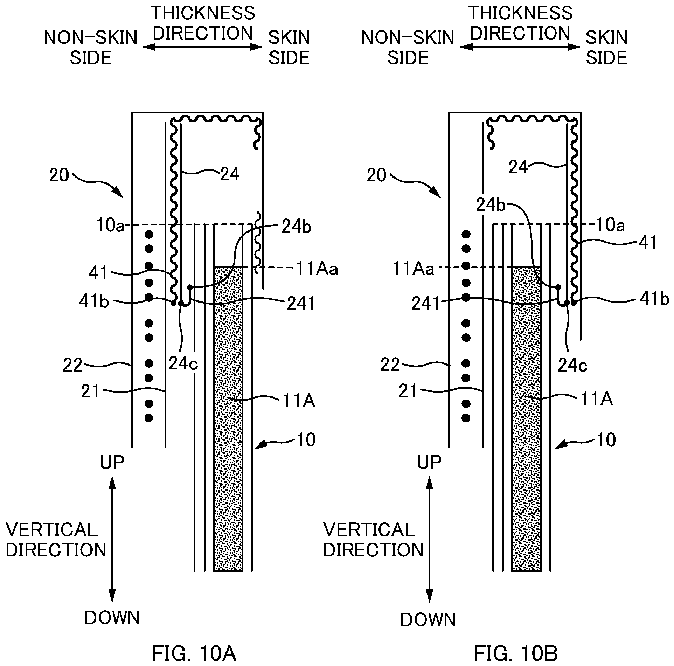

In the front waist portion 20 in FIGS. 10A and 10B, the folded portion 241 of the stretchy sheet 24 is located lower than the upper end 11Aa of the absorbent core 11A. In other words, the folded portion 241 is overlapped with the absorbent core 11A in the vertical direction.

Also, the folded portion 241 in the reference example of FIG. 10A is located on the non-skin side of the absorbent core 11A. For this reason, the folded portion 241 can bring the absorbent core 11A into close contact with the wearer from the non-skin side, and positional displacement of the absorbent main body 10 can be suppressed. Also, because the absorbent core 11A becomes heavier upon absorbing excreted fluid, the portion of the absorbent main body 10 where the absorbent core 11A is provided is the most likely to undergo positional displacement. Accordingly, in FIG. 10A, the portion of the absorbent main body 10 that is the most likely to undergo positional displacement can be brought into close contact with the wearer. This therefore makes it possible to maintain the fit of the absorbent main body 10 to the wearer and reduce the leakage of excrement.

On the other hand, the folded portion 241 in FIG. 10B is located on the skin side of the absorbent core 11A. In this case as well, the portion of the front waist portion 20 that is overlapped with portion of the absorbent main body 10 that is the most likely to undergo positional displacement can be brought into close contact with the wearer by the folded portion 241, and positional displacement of the absorbent main body 10 can be suppressed. Also, in FIG. 10B, the stretchy sheet 24 covers the skin side of the upper ends 10a and 11Aa of the absorbent main body 10 and the absorbent core 11. Accordingly, the wearer is less likely to feel the upper ends (edges) 10a and 11Aa of the absorbent main body 10 and the absorbent core 11, and it is possible to suppress the case where the wearer feels an unpleasant portion.

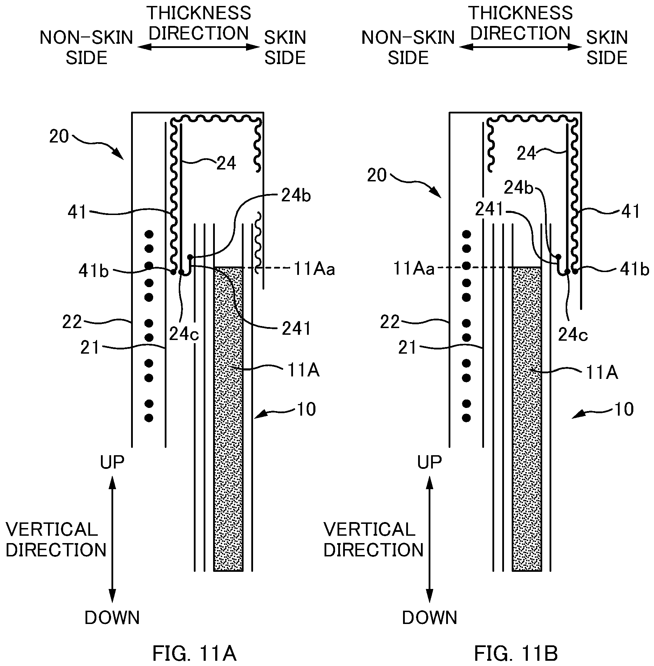

In the front waist portion 20 in FIGS. 11A and 11B, the folded portion 241 of the stretchy sheet 24 extends across the upper end 11Aa of the absorbent core 11A in the vertical direction. In this case, it is possible to gradually vary the number of material layers in the vicinity of the upper end 11Aa of the absorbent core 11A.

Specifically, the number of material layers can increase from the upper side to the lower side, that is to say, there is a region in which the upper end portion of the absorbent main body 10 and one layer of the stretchy sheet 24 are overlaid on each other, a region in which the upper end portion of the absorbent main body 10 and two layers of the stretchy sheet 24 (the folded portion 241) are overlaid on each other, and a region in which the region of the absorbent main body 10 in which the absorbent core 11A is provided and two layers of the stretchy sheet 24 are overlaid on each other. Accordingly, it is possible to reduce a height difference and a stiffness difference between the region having the absorbent core 11A and the region not having it, and it is possible to suppress the case where the wearer feels an unpleasant portion.

In particular, in FIG. 11B, the folded portion 241 of the stretchy sheet 24 covers the skin side of the upper end 11Aa of the absorbent core 11. Accordingly, even more so than in the case shown in FIG. 10B, the wearer is not likely to feel the upper end (edge) 11Aa of the absorbent core 11A, and it is possible to suppress the case where the wearer feels an unpleasant portion.

### Stretchy sheet 24 and fixing portion 41

In the product state (FIG. 5 which relates to a reference example) of the front waist portion 20, the first-end-22a-side portion of the outer layer sheet 22 has been folded downward. A length L1 is the vertical length from the upper end 20a of the front waist portion 20 to one end of the folded-down portion of the non-stretchy sheet (here, the first end 22a of the outer layer sheet 22). Also, the vertical length from the upper end 20a of the front waist portion 20 to the first end 24a of the stretchy sheet 24 is zero. It is sufficient that this length (zero) is shorter than the aforementioned length L1.

In other words, it is sufficient that the first end 24a of the stretchy sheet 24 is located higher than the first end 22a of the folded portion of the outer layer sheet 22. According to this configuration, the stretchy sheet 24 is not located too low. The fit of the upper end portion of the front waist portion 20 can thus be maintained.

Also, the fixing portion 41 has a first region 411 that fixes one surface 24A (the non-skin-side surface in FIG. 4) of the stretchy sheet 24 to the non-stretchy sheets 21 and 22, and a second region 412 that is the region excluding the first region 411. The second region 412 of the fixing portion 41 is a region of the fixing portion 41 that is continuous with the first region 411, and is provided in the non-stretchy sheet (here, the outer layer sheet 22), extending beyond the first end 24a of the stretchy sheet 24.

In the in-production state (FIG. 4 which relates to the reference example), the first region 411 of the fixing portion 41 is located between the stretchy sheet 24 and the inner layer sheet 21, and is not exposed. On the other hand, the second region 412 of the fixing portion 41 provided on the outer layer sheet 22 is exposed on the skin side. If the second region 412 of the fixing portion 41 were exposed on the skin side in the product state, hardened adhesive 41 would come into contact with the wearer, and the comfort of the front waist portion 20 would degrade. Also, although not shown, even in the case where the second region 412 of the fixing portion 41 is exposed on the non-skin side, the texture of the front waist portion 20 when touched from the outside degrades.

For this reason, it is preferable that at least a portion of the second region 412 of the fixing portion 41 is not exposed. It is more preferable that the entirety of the second region 412 of the fixing portion 41 is not exposed. In other words, it is preferable that the second region 412 of the fixing portion 41 is located between constituent materials of the diaper 1.

In this case, the second region 412 of the fixing portion 41 is covered with the folded-down portion 221 of the outer layer sheet 22 on the skin side. In other words, the second region 412 of the fixing portion 41 is located between the folded-down portion 221 of the outer layer sheet 22 and the stretchy sheet 24, and is not exposed. Accordingly, it is possible to suppress a degradation in the comfort and the texture of the front waist portion 20.

Note that the configuration of the front waist portion 20 is not limited to the configuration shown in the reference example of FIG. 5. Other examples include a configuration in which the inner layer sheet 21 is also folded downward along with the outer layer sheet 22, a configuration in which only the inner layer sheet 21 is folded downward, and a configuration in which both the inner layer sheet 21 and the outer layer sheet 22 are not folded downward. Accordingly, the material that covers the skin side of the second region 412 of the fixing portion 41 is not limited to being the outer layer sheet 22. Other examples include a folded-down portion of the inner layer sheet 21 and a sheet other than the non-stretchy sheets 21 and 22.

Also, in the product state (FIG. 5 which relates to the reference example), the first-end-41a-side portion of the fixing portion 41 has been folded downward. In other words, the method for manufacturing the diaper 1 includes a step of folding the fixing portion 41 downward along with the outer layer sheet 22.

For this reason, the fixing portion 41 is overlaid on the upper end portion of the front waist portion 20 in the thickness direction. More specifically, there are two layers of the adhesive 41, and the stiffness is higher in the upper end portion of the front waist portion 20. Accordingly, when the diaper 1 is put on, the upper end portion of the front waist portion 20 can be grabbed more easily, and the diaper 1 can be pulled up more easily. It is also possible to suppress the case where the upper end portion of the front waist portion 20 curls and becomes creased during packaging or the like, thus suppressing a degradation in the appearance of the diaper 1.

Also, although not shown, the first end 24a of the stretchy sheet 24 may be located lower than the upper end 20a of the front waist portion 20. In this case, it is sufficient that the first-end-41a-side portion of the fixing portion 41 is not folded downward, and the first end 41a of the fixing portion 41 is located higher than the first end 24a of the stretchy sheet 24. According to this configuration, curling of the first-end-24a-side portion of the stretchy sheet 24 is suppressed, there is one layer of the fixing portion 41 in the upper end portion of the front waist portion 20, and the upper end portion of the front waist portion 20 is softer.

Furthermore, the first end 41a of the fixing portion 41 may also be located lower than the upper end 20a of the front waist portion 20. In this case, the upper end portion of the front waist portion 20 has a region in which the stretchy sheet 24 and the fixing portion 41 are not provided. The upper end portion of the front waist portion 20 is therefore softer.

If the upper end portion of the front waist portion 20 is softer, it is possible to prevent irritation of the wearer's skin by the upper end (edge) 20a of the front waist portion 20. Also, in the natural state of the diaper 1, the upper end portion of the front waist portion 20 is likely to have a frilly shape. It is therefore possible to give the user the impression that the diaper 1 is soft and comfortable.

### Other embodiments

Although the embodiment of the present disclosure has been described hereinabove, the above embodiment of the present disclosure is simply to facilitate understanding of the present disclosure and are not in any way to be construed as limiting the present disclosure. The present disclosure may variously be changed or altered without departing from the scope of the invention as defined by claims.

For example, the stretchy sheet 24 is not limited to being provided in the upper end portion of the front waist portion 20. The stretchy sheet 24 may be provided at any position in the front waist portion 20 in the vertical direction. Also, a configuration is possible in which the back waist portion 30 (first waist portion) has a stretchy sheet, and the front waist portion 20 (second waist portion) does not have a stretchy sheet, or the front waist portion 20 and the back waist portion 30 may both have the stretchy sheet 24.

Also, although a diaper in which the front waist portion 20 and the back waist portion 30 are separate members is described as an example in the above embodiment, a configuration is possible in which the crotch portion is provided between the front waist portion 20 and the back waist portion 30, and the front waist portion 20 and the back waist portion 30 are a single continuous member.

### [Reference Signs List]

1 diaper (absorbent article), 2 joining region,
10 absorbent main body, 11 absorbent body,
11A absorbent core, 11B core-wrapping sheet,
12 top sheet, 13 back sheet,
14 exterior sheet, 15 leg elastic member,
20 front waist portion (first waist portion),
21 inner layer sheet (non-stretchy sheet), 22 outer layer sheet (non-stretchy sheet),
23 string-like elastic member, 24 stretchy sheet,
30 back waist portion (second waist portion), 31 inner layer sheet,
32 outer layer sheet, 33 string-like elastic member,
41 fixing portion (adhesive), 42 adhesive

## Claims

1. An absorbent article (1) having a vertical direction and a lateral direction,
the absorbent article comprising:
an absorbent main body (10);
a first waist portion (20); and
a second waist portion (30),
the first waist portion (20) including a stretchy sheet (24) and a non-stretchy sheet (21,22),
the stretchy sheet (24) being capable of stretching and contracting in the lateral direction,
the non-stretchy sheet (21, 22) being overlaid on the stretchy sheet,
a lower end portion of the stretchy sheet (24) being folded upward,
a number of layers of the stretchy sheet being greater in the lower end portion of the stretchy sheet (24) than in an upper end portion of the stretchy sheet,
the stretchy sheet (24) being located on a skin side in a thickness direction of the first waist portion (20) with respect to the absorbent main body (10), and
in the lower end portion of the stretchy sheet,
a folded portion of the stretchy sheet (24) being overlapped with the absorbent main body (10) in the vertical direction.

2. The absorbent article according to claim 1, wherein
the absorbent main body (10) includes an absorbent core (11A) configured to absorb an excreted fluid, and
the folded portion of the stretchy sheet (24) is not overlapped with the absorbent core (11A) in the vertical direction.

3. The absorbent article according to claim 1 or 2, wherein
the folded portion of the stretchy sheet (24) is provided extending across an upper end of the absorbent main body (10) in the vertical direction.

4. The absorbent article according to claim 1, wherein
the absorbent main body (10) includes an absorbent core (11A) configured to absorb an excreted fluid, and
the folded portion of the stretchy sheet (24) is overlapped with the absorbent core (11A) in the vertical direction.

5. The absorbent article according to claim 4, wherein
the folded portion of the stretchy sheet (24) is provided extending across an upper end of the absorbent core (11A) in the vertical direction.

6. The absorbent article according to any one of claims 1 to 5, wherein
in a state where the lower end portion of the stretchy sheet (24) is folded back,
two lateral side end portions of the first waist portion (20) are welded to two lateral side end portions of the second waist portion (30).

## Patentansprüche

1. Absorbierender Artikel (1), der eine vertikale Richtung und eine laterale Richtung aufweist,
wobei der absorbierende Artikel Folgendes umfasst:
einen absorbierenden Hauptkörper (10);
ein erstes Taillenteil (20); und
ein zweites Taillenteil (30),
wobei das erste Taillenteil (20) eine dehnbare Lage (24) und eine nicht-dehnbare Lage (21, 22) einschließt,
sich die dehnbare Lage (24) in der lateralen Richtung dehnen und zusammenziehen kann,
die nicht-dehnbare Lage (21, 22) die dehnbare Lage überlagert,
ein unteres Endteil der dehnbaren Lage (24) nach oben gefaltet ist,
etliche Schichten der dehnbaren Lage größer sind in dem unteren Endteil der dehnbaren Lage (24) als in einem oberen Endteil der dehnbaren Lage,
wobei sich die dehnbare Lage (24) auf einer Hautseite in einer Dickenrichtung des ersten Taillenteils (20) in Bezug auf den absorbierenden Hauptkörper (10) befindet und
in dem unteren Endteil der dehnbaren Lage
ein gefaltetes Teil der dehnbaren Lage (24) mit dem absorbierenden Hauptkörper (10) in der vertikalen Richtung überlappt ist.

2. Absorbierender Artikel nach Anspruch 1, wobei
der absorbierende Hauptkörper (10) einen Saugkern (11A) einschließt, der zur Absorbierung einer ausgeschiedenen Flüssigkeit konfiguriert ist, und
das gefaltete Teil der dehnbaren Lage (24) nicht mit dem Saugkern (11A) in der vertikalen Richtung überlappt.

3. Absorbierender Artikel nach Anspruch 1 oder 2, wobei
das gefaltete Teil der dehnbaren Lage (24) bereitgestellt ist, dass es sich über ein oberes Ende des absorbierenden Hauptkörpers (10) in der vertikalen Richtung erstreckt.

4. Absorbierender Artikel nach Anspruch 1, wobei
der absorbierende Hauptkörper (10) einen Saugkern (11A) einschließt, der zur Absorbierung einer ausgeschiedenen Flüssigkeit konfiguriert ist, und
das gefaltete Teil der dehnbaren Lage (24) mit dem Saugkern (11A) in der vertikalen Richtung überlappt.

5. Absorbierender Artikel nach Anspruch 4, wobei
das gefaltete Teil der dehnbaren Lage (24) bereitgestellt ist, dass es sich über ein oberes Ende des Saugkerns (11A) in der vertikalen Richtung erstreckt.

6. Absorbierender Artikel nach einem der Ansprüche 1 bis 5, wobei
in einem Zustand, wo das untere Endteil der dehnbaren Lage (24) zurückgefaltet ist,
zwei laterale Seitenendteile des ersten Taillenteils (20) mit zwei lateralen Seitenendteilen des zweiten Taillenteils (30) verschweißt sind.

## Revendications

1. Article absorbant (1) ayant une direction allant dans le sens de la verticale et une direction allant dans le sens latéral,
l'article absorbant comportant :
un corps principal absorbant (10) ;
une première partie au niveau de la taille (20) ; et
une deuxième partie au niveau de la taille (30),
la première partie au niveau de la taille (20) comprenant une feuille extensible (24) et une feuille non extensible (21, 22),
la feuille extensible (24) étant en mesure de s'étendre et de se contracter dans la direction allant dans le sens latéral,
la feuille non extensible (21, 22) étant superposée sur la feuille extensible,
une partie d'extrémité inférieure de la feuille extensible (24) étant repliée vers le haut,
un certain nombre de couches de la feuille extensible étant supérieur dans la partie d'extrémité inférieure de la feuille extensible (24) que dans une partie d'extrémité supérieure de la feuille extensible,
la feuille extensible (24) étant située sur un côté peau dans une direction allant dans le sens de l'épaisseur de la première partie au niveau de la taille (20) par rapport au corps principal absorbant (10), et
dans la partie d'extrémité inférieure de la feuille extensible,
une partie repliée de la feuille extensible (24) étant chevauchée par le corps principal absorbant (10) dans la direction allant dans le sens de la verticale.

2. Article absorbant selon la revendication 1, dans lequel
le corps principal absorbant (10) comprend une partie centrale absorbante (11A) configurée pour absorber un fluide excrété, et
la partie repliée de la feuille extensible (24) n'est pas chevauchée par la partie centrale absorbante (11A) dans la direction allant dans le sens de la verticale.

3. Article absorbant selon la revendication 1 ou la revendication 2, dans lequel
la partie repliée de la feuille extensible (24) est mise en œuvre pour s'étendre en travers d'une extrémité supérieure du corps principal absorbant (10) dans la direction allant dans le sens de la verticale.

4. Article absorbant selon la revendication 1, dans lequel
le corps principal absorbant (10) comprend une partie centrale absorbante (11A) configurée pour absorber un fluide excrété, et
la partie repliée de la feuille extensible (24) est chevauchée par la partie centrale absorbante (11A) dans la direction allant dans le sens de la verticale.

5. Article absorbant selon la revendication 4, dans lequel
la partie repliée de la feuille extensible (24) est mise en œuvre pour s'étendre en travers d'une extrémité supérieure de la partie centrale absorbante (11A) dans la direction allant dans le sens de la verticale.

6. Article absorbant selon l'une quelconque des revendications 1 à 5, dans lequel
dans un état dans lequel la partie d'extrémité inférieure de la feuille extensible (24) est repliée,
deux parties d'extrémité côté latéral de la première partie au niveau de la taille (20) sont soudées à deux parties d'extrémité côté latéral de la deuxième partie au niveau de la taille (30).
